# EUROPEAN PATENT APPLICATION

(11) **EP 4 332 428 A1**
(43) Date of publication of application: **06.03.2024**
(21) Application number: 22193078.7
(22) Date of filing: 31.08.2022
(51) Int. Cl.: F21S 8/02, F21V 33/00, F21V 29/67, A61L 9/20, F21Y 115/10, F21Y 113/00

(54) **DOWNLIGHT WITH STERILIZATION FUNCTIONALITY**

(71) Applicant: Signify Holding B.V., 5656 AE Eindhoven (NL)
(72) Inventor: The designation of the inventor has not yet been filed
(74) Representative: Verweij, Petronella Daniëlle

(57) **Abstract**

A downlight lamp that provides both visible light illumination and air sterilization. Unsterilized air is drawn through one or more air inlet channels, sterilized by a sterilization device and expelled through one or more air outlet channels. The movement of the air is controllable by a fan. The opening(s) of the air inlet channel(s) are angled with respect to the opening(s) of the air outlet channel(s). The openings are positioned in a protruding portion of the downlight lamp, being a portion that protrudes outwardly from a supporting surface in a same direction as visible light is emitted.

## Description

### FIELD OF THE INVENTION

The present invention relates to the field of downlights, and in particular to downlights capable of disinfecting or sterilizing air.

### BACKGROUND OF THE INVENTION

As transmissible diseases, such as COVID-19, are becoming more common and widespread throughout the word, there is an increasing interest in the development of disinfection or sterilization devices.

One particular area of interest is to provide downlight lamps or lighting devices that are able to illuminate the environment as well as perform a disinfection or sterilization role. Such products benefit from reduced requirements for power sockets, as existing sockets can be repurposed for a dual-function. It is also recognized that lamp sockets are naturally well placed to perform sterilization, as lamp sockets are typically positioned in order to illuminate a large area or areas in which humans are most likely to go.

There is an increasing interest in improving the efficiency and effectiveness of downlight lamps that are able to perform this dual role of illumination and sterilization.

### SUMMARY OF THE INVENTION

The invention is defined by the claims.

According to examples in accordance with an aspect of the invention, there is provided a downlight lamp for providing visible light illumination and air sterilization. The downlight lamp is adapted for mounting on a supporting member and comprises: a visible light arrangement for emitting visible light out of the downlight lamp; one or more air inlet channels for receiving unsterilized air into the downlight lamp, each air inlet channel comprising an air inlet opening through which air is received into the downlight lamp; one or more air outlet channels for outputting sterilized air from the downlight lamp, each air outlet channel comprising an air outlet opening through which air is output from the downlight lamp; a sterilization chamber coupled to the one or more air inlet channels and the one or more air outlet channels; a sterilization device configured to sterilize air in the sterilization chamber; and a fan for moving air through the one or air inlet channels and out of the one or more air outlet channels via the sterilization chamber.

The downlight lamp is configured wherein any plane in which any air inlet opening lies makes an angle of between 60° and 120° to any plane in which any air outlet opening lies; and wherein each air inlet opening and each air outlet opening are provided at a portion of the downlight lamp that protrudes from a side of the supporting member in the same direction as the visible light is emitted out of the downlight lamp.

The proposed downlight lamp thereby provides a system that is able to provide both visible light illumination and sterilization or disinfection of air. Air is moved through one or more air inlet channels, to a sterilization chamber for sterilization, and out of one or more air outlet channels. Air enters the air inlet channel(s) through air inlet opening(s) and air exits the air outlet channel(s) through air outlet opening(s). The air inlet opening(s), used to transport unsterilized air into the lamp, and the air outlet opening(s), used to transport sterilized air out of the lamp, are angled with respect to one another. The angle is between 60° and 120°.

One advantage of this approach is to reduce recirculation of sterilized air, as the sterilized air will be expelled in a direction that is angled with respect to the direction of air being taken into the downlight lamp, i.e., away from the air inlet channel(s) or air inlet openings. This reduces the likelihood that the sterilized air will be taken back into the lamp.

Another advantage of this approach is to improve the distribution of sterilized air. Angling the air inlet opening(s) and air outlet opening(s) avoids the downlight lamp from simply sterilizing a narrow column of air, instead circulating the sterilized air across a larger area or volume.

In normal cases, downlights are mounted with its front rim flush with the mounting surface, e.g., a ceiling. However, in this invention, the opening(s) is/are provided on a protruding portion, which exposes the opening(s) to a greater amount of air. This improves the efficiency and effectiveness of the downlight lamp, by allowing a greater volume of unsterilized air to be present in the vicinity of the downlight lamp.

In some embodiments, any plane in which any air inlet opening lies makes an angle of between 80° and 100°, and preferably between 85° and 95°, to any plane in which any air outlet opening lies. This approach further avoids the recirculation of the sterilized air and improves the distribution of sterilized air.

In some examples, the shape of each air inlet channel and each air outlet channel is curved. This embodiment reduces a line of sight or field of view of the sterilization device from outside the lamp. This improves the visual appearance of the downlight lamp, and reduces a chance of undesirable sterilization (e.g., ultraviolet radiation) from escaping the downlight lamp.

In some embodiments, each air inlet channel is positioned between at least one air outlet channel and the visible light arrangement. This approach causes sterilized air to be expelled away from the air inlet channels, making the air less likely to simply be recirculated within the downlight lamp.

In some examples, each air outlet opening is positioned to point away from each air inlet opening. This approach also causes sterilized air to be expelled away from the air inlet channels, making the air less likely to be recirculated within the downlight lamp.

In some examples, the visible light arrangement comprises: a visible light emitter configured to generate and emit visible light; and a visible light reflector configured to direct any visible light generated by the visible light emitter out of the downlight lamp, wherein the visible light reflector provides at least part of the bounds or boundaries of at least one air inlet channel and/or air outlet channel.

The downlight lamp may further comprise a first sleeve surrounding the visible light reflector, wherein the space between the first sleeve and the visible light reflector defines either: the one or more air inlet channels or the one or more air outlet channels; and a second sleeve surrounding the first sleeve, wherein the space between the first sleeve and the second sleeve defines the other of: the one or more air inlet channels or the one or more air outlet channels. This approach provides a compact and space-efficient approach for permitting air flow within the downlight lamp for performing sterilization.

The fan may comprise a centrifugal fan. A centrifugal fan provides a more compact device or allows larger sizes of air inlet channels and/or air outlet channels. This is because the need for an air inlet channel/outlet to extend from a rear of the fan (and back to the front to head in a direction for being emitted from the lamp) is avoided. Rather, the air inlet channel/outlet can extend to a side of the fan.

Preferably, the sterilization device comprises an ultraviolet light emitter configured to irradiate the sterilization chamber with ultraviolet light to sterilize or disinfect air in the sterilization chamber. This provides a cheap and compact mechanism for performing sterilization of air.

The ultraviolet light emitter is configured to only emit ultraviolet light in directions that neither head toward the one or more air inlet channels nor toward the one or more air outlet channels. Thus, the directions do not head toward the one or more air inlet channels and/or one or more air outlet channels. This approach reduces the amount of ultraviolet light that will exit the air inlet channel and/or air outlet channel, improving a safety to individuals in the region of the downlight lamp.

The downlight lamp may further comprise one or more baffles positioned to at least partially shield the at least one air outlet opening. This embodiment reduces a line of sight or field of view of the sterilization device from outside the lamp. This improves the visual appearance of the downlight lamp, and reduces a chance of undesirable sterilization (e.g., ultraviolet radiation) from escaping the downlight lamp. The baffles may be alternatively named louver or fins.

For embodiments where the sterilization device comprises an ultraviolet light emitter, this approach also reduces or blocks at least some of the ultraviolet light from escaping or being output from the downlight lamp, without significantly impacting on the ability of the lamp to draw and/or expel air. This improves a safety of the downlight lamp.

In some examples, the downlight lamp comprises one or more baffles positioned to at least partially shield the at least one air inlet opening.

In some examples, the width of any of the air outlet opening is greater than the width of any air inlet opening. In particular, the ratio between the width of any air outlet opening and any air inlet opening may be greater than 1.2, e.g., 1.6.

In some examples, the cross-sectional area of the air outlet opening(s) is greater than the cross-sectional area of the air inlet opening(s).

In some embodiments, the average cross-sectional area of any air outlet channel is greater than the average cross sectional area of any air inlet channel.

In some examples, the sterilization device is positioned between the one or more air inlet channels and the fan. This approach avoids or reducing the likelihood of unsterilized air from reaching the fan, reducing the likelihood of mixing of unsterilized and sterilized air and improving the efficiency of the downlight lamp.

Optionally, the downlight lamp comprises: a first end for connecting to a lamp socket; and a second end at which visible light, emitted by the visible light arrangement, is output, wherein each air inlet opening and each air outlet opening are positioned at the second end. This approach provides a more compact and efficient downlight lamp. The sterilized air will be output into the same environment as that which is illuminated by the visible light arrangement. The protruding portion is provided at the second end.

The visible light arrangement may be configured to emit light having a wavelength greater than 380nm.

Preferably, the light having a wavelength greater than 380nm includes light having a wavelength greater than 500nm. Thus, the visible light arrangement may output light that includes at least green, yellow, orange and/or red light. For instance, the visible light arrangement may be configured to emit white light.

Put another way, the visible light arrangement may be configured to emit light that includes light having a wavelength of between 380nm and 500nm and light having a wavelength greater than 500nm.

It will be clear that the visible light arrangement and the sterilization device form separate elements of the downlight lamp.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 provides a cross-sectional view of a downlight lamp;
Fig. 2 provides a more detailed cross-sectional view of a downlight lamp;
Fig. 3 illustrates a downlight lamp installation;
Fig. 4 provides an exploded view of a downlight lamp; and
Fig. 5 provides a side view of a downlight lamp.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Proposed embodiments provide a downlight lamp that provides both visible light illumination and air sterilization. Unsterilized air is drawn through one or more air inlet channels, sterilized by a sterilization device and expelled through one or more air outlet channels. The movement of the air is controllable by a fan. The opening(s) of the air inlet channel(s) are angled with respect to the opening(s) of the air outlet channel(s). The openings are positioned in a protruding portion of the downlight lamp, being a portion that protrudes outwardly from a supporting surface in a same direction as visible light is emitted.

Embodiments are based on the realization that angling the air inlet opening(s) to the air outlet opening(s) can reduce recirculation of air within the downlight lamp, thereby providing more efficient sterilization. Efficiency can be further enhanced by positioning the openings in a protruding portion, as this exposes the openings to a greater volume and/or amount of air, reducing the likelihood of recirculation.

Approaches can be employed in any environment in which sterilization of air is desired, for instance, in home, office, clinical and/or industrial environments. Approaches are particularly advantageous in areas comprising a large number of lamps (e.g., office environments), as the proposed approach provides efficient sterilization of regions in the vicinity of lamps.

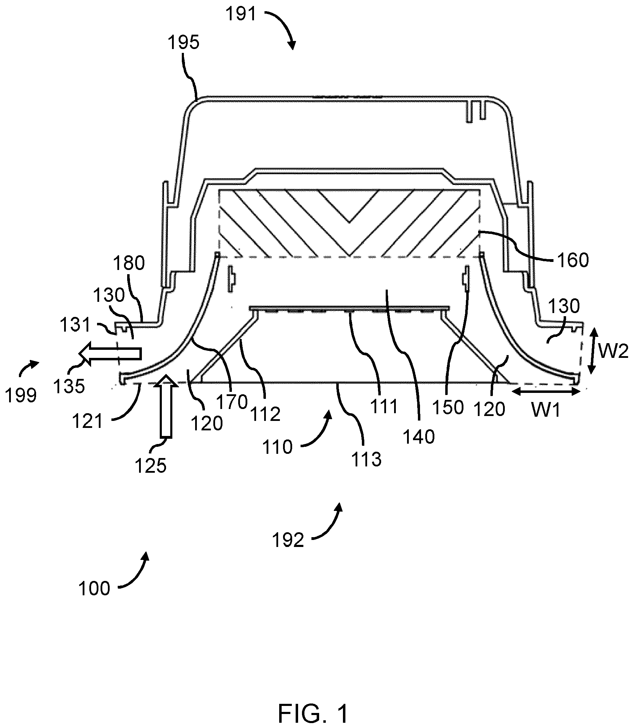

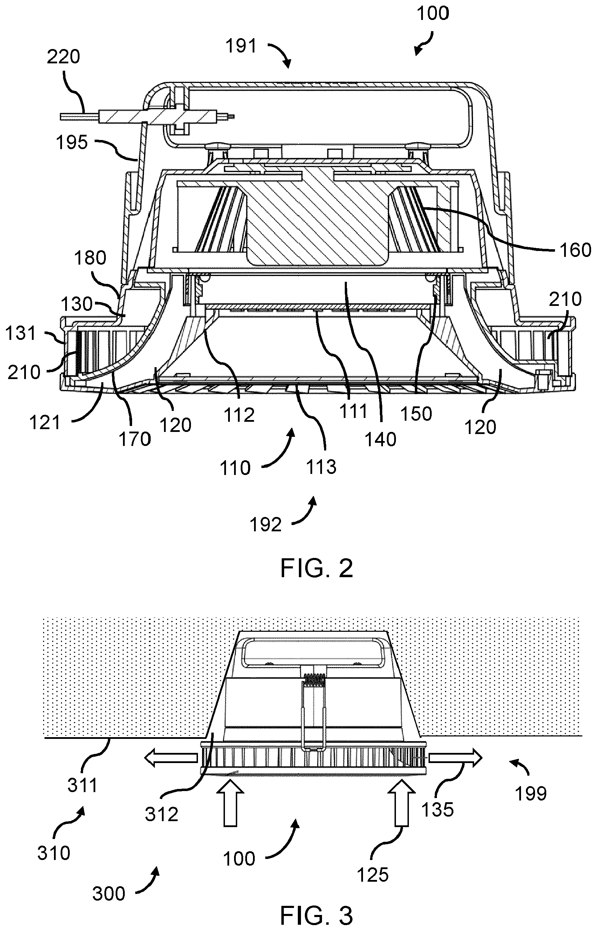

Figures 1 and 2 provide cross-sectional views of a downlight lamp 100 according to an embodiment. Figure 1 illustrates the position of the more significant features of the downlight lamp, with Figure 2 provide a more detailed illustration of an overall embodiment.

The downlight lamp 100 comprises a visible light arrangement 110 that is configured to emit light out of the downlight lamp.

The illustrated visible light arrangement 110 comprises a visible light emitter 111 configured to generate and emit visible light. The visible light emitter 111 may comprise one or more LEDs. Preferably, the visible light emitter is configured to emit light having a wavelength greater than 380nm. This preferably includes light having a wavelength greater than 500nm, e.g., greater than 550nm. Of course, the visible light emitter may also emit light having lower wavelengths, e.g., wavelengths between 380nm and 500nm, e.g. wavelength between 400nm and 500nm. For instance, the visible light emitter may be configured to emit white light containing a broad spectrum of wavelengths.

The illustrated visible light arrangement 110 also comprises a visible light reflector 112. The visible light reflector is configured to direct any visible light generated by the visible light emitter out of the downlight lamp. The visible light reflector may have a generally conical shape, e.g., a frustum shape, for improved directing of visible light out of the lamp 100.

The visible light arrangement 110 may comprise a lens 113 or protective cover. If configured as a lens, the lens may be configured to focus/shape or scatter/diffuse light emitted by the visible light emitter when exiting the downlight lamp. A protective cover protects the visible light emitter from the ingress of dust or interference (e.g., by a person). Of course, a lens 113 is also able to act as a protective cover. The lens 113 or protective cover is formed from transparent and/or translucent material to permit the passage of visible light, generated by the visible light emitter, out of the lamp 100.

The downlight lamp 100 also comprises one or more air inlet channels 120. Each air inlet channel receives unsterilized air 125 into the downlight lamp through an air inlet opening 121 of the air inlet channel 120.

The downlight lamp also comprises one or more air outlet channels 130. Each air outlet channel is configured to expel or output sterilized air 135 from the downlight lamp through an air outlet opening 131 of the air outlet channel.

Each air inlet channel 120 and air outlet channel 130 is therefore a passage, duct or conduit that permits the flow of gas (i.e., air) therethrough. In some examples, the air inlet channel(s) is/are configured to completely surround or encircle the visible light arrangement 110. Similarly, in some examples, the air outlet channel(s) is/are configured to completely surround or encircle the visible light arrangement.

Each air inlet opening 121 and air outlet opening 131 is an aperture, hole or gap through which air 125, 135 is able to enter or exit the air inlet channel 120 and air outlet channel 130 respectively.

The air inlet channel(s) and/or air outlet channel(s) may be formed of any suitable material for transporting air, e.g., plastics or metals.

The sterilized air 135 is produced in a sterilization chamber 140, which is fluidly coupled to/between the air inlet channel(s) 120 and the air outlet channel(s) 130. Thus, unsterilized air 125 is able to move through the air inlet channel 120 to the sterilization chamber 140 where it is sterilized. The sterilized air 135 then moves through the air outlet channel(s) 130 and is output or expelled from the downlight lamp through the air outlet opening(s) 131.

The downlight lamp 100 comprises a sterilization device 150 to perform the sterilization of the air in the sterilization chamber 140, i.e., to produce the sterilized air. The sterilization device 150 may, for instance, be housed in the sterilization chamber 140. Examples of sterilization devices 150 are well known to the skilled person.

The visible light arrangement and the sterilization device are formed in/of different chambers of the downlight lamp.

One example of a sterilization device is a heater, configured to heat the air in the sterilization chamber 140 to perform disinfection or sterilization. This is less preferred, as heating may damage or affect the other components of the sterilization device, e.g., the light emitting arrangement.

A particularly advantageous example of a sterilization device is an ultraviolet light emitter, which is configured to irradiate the sterilization chamber 140 with ultraviolet light to sterilize air in the sterilization chamber. Examples of ultraviolet light emitters for performing sterilization or disinfection are well known in the art, e.g., ultraviolet light emitters that emit light in the UV-C spectrum (e.g., having a wavelength of around 260-280nm).

The ultraviolet light emitter may be configured to only emit ultraviolet light in directions that neither head toward the one or more air inlet channels nor toward the one or more air outlet channels, i.e., in directions that do not head toward the one or more air inlet channels and/or one or more air outlet channels. Thus, the ultraviolet light emitter may be configured to emit ultraviolet light in a direction away from a portion of the lamp comprising or containing the air inlet channel(s) and/or air outlet channel(s). This reduces the chances of ultraviolet light exiting or escaping the sterilization chamber and/or the downlight lamp.

In one configuration, the ultraviolet light emitter comprises one or more UV-C LEDs, such as UV-C LEDs that output a light having a 30° beam angle. This approach decreases the minimum sterilization chamber size, e.g., compared to conventional UV-C light tubes. The UV-C LEDs can also provide more concentrated radiation energy, which is better for killing viruses and/or bacteria.

To move the unsterilized and sterilized air through the downlight lamp 100, the downlight lamp 100 comprises a fan 160. The fan 160 is configured to (e.g., controllably) move air through the one or air inlet channels 120 and out of the one or more air outlet channels 130 via the sterilization chamber 140.

More particularly, in the illustrated example, the fan 160 moves the unsterilized air through the air inlet channel(s) 120 to the sterilization chamber 140, where the air is sterilized by the sterilization device 150. The sterilized air is then moved, by the fan 160, through the fan and to the air outlet channel(s) 130. The fan 160 pushes the sterilized air through the air outlet channel(s) and expels the air out of downlight lamp via the air outlet opening(s) 131.

It will be appreciated that the precise arrangement of components of the downlight lamp can be modified. For instance, the sterilization chamber could be instead positioned between the fan 160 and the air outlet channel(s) 130. This would still achieve a same function of sterilizing and circulating air.

The fan 160 may comprise a centrifugal fan. Use of a centrifugal fan provides a more compact device and allows larger sizes of air inlet channel(s) 120 and/or air outlet channel(s) 130. In particular, the centrifugal fan 160 can be used to rotate or change the direction of the air and to drive the air to get more air flow volume (e.g., compared to approaches that do not make use of a fan).

The downlight lamp 100 is configured such that any plane in which any air inlet opening 121 lies makes an angle of between 60° and 120°, e.g., between 80° and 100° or between 85° and 95°, with respect to any plane in which any air outlet opening 131 lies. This approach reduces a likelihood that sterilized air expelled from the air outlet opening(s) is simply redrawn into the air inlet opening(s). This improves the efficiency of the overall sterilization performed by the downlight lamp, as more air is sterilized by the downlight lamp over a period of time.

The downlight lamp 100 is also configured such that each air inlet opening 121 and each air outlet opening 131 are provided at a protruding portion 199 of the downlight lamp 100. The protruding portion 199 is a portion of the downlight lamp 100 that protrudes from a side of a supporting member (to which the downlight lamp is mounted) in a same direction as which the visible light is emitted out of the downlight lamp.

Put another way, it is possible to define a first end 191 of the downlight lamp for connecting the lamp to a lamp socket or supporting member; and a second end 192 from which visible light, emitted by the visible light arrangement, is output. The protruding portion 199 is located at the second end 192 of the downlight lamp. The protruding portion 199 may be configured to protrude outwardly from the supporting member when the downlight lamp is mounted to the supporting member.

The configuration of the protruding portion 199 is perhaps best illustrated by Figure 3, which illustrates a downlight lamp installation 300 in which a downlight lamp is mounted on a supporting member 310, e.g., a ceiling, a floor or a wall.

The supporting member 410 defines a side 311. The protruding portion 199 protrudes from the side 311 in a same direction as the visible light is emitted from the downlight lamp 100.

If the supporting member 310 comprises a recess 312, e.g., for receiving the downlight lamp, the protruding portion 199 is not held or included within the recess 312.

Turning back to Figures 1 and 2, the air inlet channel(s) 120 and/or air outlet channel(s) 130 may be curved in shape and/or comprise a curved portion. In this way, the air inlet channel(s) and/or air outlet channel(s) may bend or (at least partially) turn back on itself.

This approach reduces the size of any direct line of sight from outside the downlight lamp 100 to the sterilization chamber 140 and/or the sterilization device. In particular, the curvature of the air inlet channel(s) and/or air outlet channel(s) reduces the amount of light that can travel in a straight line from the sterilization chamber and/or sterilization device to outside of the lamp 100.

This technique is particularly advantageous when the sterilization device 150 comprises an ultraviolet light emitter. In particular, light output by the ultraviolet light emitter will be at least partially absorbed by the walls or sides air inlet channel(s) 120 and/or air outlet channel(s) 130, reducing the exposure of an external environment to the lamp (e.g., persons located outside of the lamp 100) to ultraviolet light and/or visible light (e.g., blue light) produced by the ultraviolet light emitter.

Another approach for reducing the size of any direct light of sight from outside the downlight lamp 100 to the sterilization chamber 140 is the use of one or more baffles 210 or louver positioned to at least partially shield or obscure the air inlet channel(s) 120 and/or air outlet channel(s) 130. A baffle 210 or louver is a slat or fin that interrupts a line of sight from outside the lamp 100 to the sterilization chamber 140 through the air inlet channel(s) 120 and/or air outlet channel(s) 130.

This technique is also advantageous when the sterilization device 150 comprises an ultraviolet light emitter, as the baffle(s) or louvre will act to block any hazard light escaping the downlight lamp 100.

The baffles 210 may be configured and/or positioned to partially overlap one another, e.g., to complete block a line of sight to the air outlet channel(s) and/or air inlet channel(s) from a position external to the downlight lamp. The overlapping is configured to provide a path for air to pass. This approach further reduces the view or field of view of sterilization chamber without significantly affecting the passage of air, further improving the safety of the downlight lamp 100 without significantly impacting on the sterilization efficiency.

By way of example, the baffles 210 may be positioned and angled to block the line of sight to the air inlet channel(s) and/or air outlet channel(s).

In the illustrated example, each air inlet channel 120 is positioned between at least one air outlet channel 130 and the visible light arrangement 110. In particular, the air outlet channel(s) is/are configured to completely surround or encircle the air inlet channel(s).

However, in other arrangements, each air outlet channel 130 may be positioned between at least one air inlet channel 120 and the visible light arrangement 110. In particular, the air inlet channel(s) may be configured to completely surround or encircle the air inlet channel(s).

The downlight lamp 100 may comprise a first sleeve 170 surrounding the visible light reflector 112 and a second sleeve 180 surrounding the first sleeve. A space between the first sleeve and the visible light reflector 112 can define the air inlet channel(s) or the air outlet channel(s). Similarly, the space between the first sleeve 170 and the second sleeve 180 can define the other of the air inlet channel(s) and the air outlet channel(s).

This configuration provides a compact arrangement for defining the flow of air through the downlight lamp.

This configuration is particularly advantageous when the fan 160 comprises a centrifugal fan, as sterilized air can be output sidewards by the fan 160 directly into the air outlet channel(s), avoiding a need for a lengthy passage for the air outlet channel and/or allowing for fans with greater air flow rates to be used.

Under this configuration, the centrifugal fan would draw the unsterilized air 125 in through the air inlet opening 121 and through the air inlet channel 120, which is formed between or bounded by the visible light reflector 111 and the first sleeve 170. The unsterilized air then arrives at the sterilization chamber 140, which is formed or located between the visible light reflector 112 and the fan 160. After disinfection or sterilization by the sterilization device 150 in this chamber, the sterilized air 135 is drawn in by the centrifugal fan and blown into the air outlet channel(s) 130, which is/are formed between or bounded by the first sleeve and the second sleeve. Finally, the sterilized air 135 is expelled from the air outlet opening 131.

Of course, the direction of the centrifugal fan could be reversed. This would effectively swap the air inlet channel(s) and air outlet channel(s) over.

The downlight lamp 100 may comprise a housing 195. The housing is configured to couple to and support the other elements of the downlight lamp. In the illustrated example, the housing 195 couples to the second sleeve 180. The housing 195 may be the portion of the downlight lamp that couples to the supporting member, e.g., mounts the downlight lamp 100.

The visible light arrangement 110 may be configured such that emitted visible light is not directed towards the sterilization chamber 140. This can be achieved, for instance, by configuring the visible light reflector 111 to reflect or direct visible light away from the sterilization chamber, e.g., out of the device. This can be achieved through the use of suitable reflective materials.

If the sterilization device 150 is an ultraviolet light emitter, the sterilization chamber 140 may be configured to retain emitted ultraviolet light within the sterilization chamber. This can be achieved through the use of reflective materials to form the sides of the sterilization chamber.

The sterilization chamber 140 may be stacked on the visible light arrangement 110, such that one or more boundaries, bounds, surfaces or sides of the visible light arrangement 110 define at least some of the boundaries/bounds of the sterilization chamber 150. Surfaces of the visible light arrangement that bound the sterilization chamber 150 may, for instance, be reflective or coated in a reflective material to improve (if present) the retention of ultraviolet light within the sterilization chamber 150.

As best illustrated in Figure 2, the downlight lamp 100 may comprise a power connector 220 for connecting the downlight lamp to a power source such as a mains supply. Other features and elements required for powering a downlight lamp may also be provided (e.g., a driver, power converter and/or power storage system), but are well known to the skilled person and are not illustrated for the sake of conciseness.

The dimensions of the air inlet opening(s) and or air outlet opening(s) may be configured such the air outlet opening(s) is/are larger than the air inlet opening(s). Thus, a ratio W2/W1 of the width W1 of the air inlet opening to the width W2 of the air outlet opening may be greater than 1.2.

In one example, the ratio W2/W1 is 1.6.

In some examples, the cross-sectional area of the air outlet opening(s) is greater than the cross-sectional area of the air inlet opening(s). This can be achieved through appropriate configuration of the channels and/or openings.

In some embodiments, the average cross-sectional area of any air outlet channel is greater than the average cross sectional area of any air inlet channel. This can be achieved through appropriate configuration of the channels.

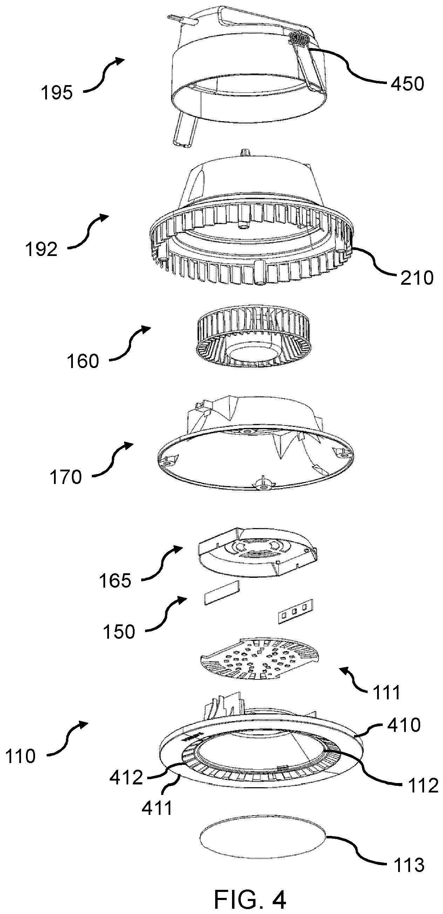

Figure 4 provides an exploded view of the downlight lamp, for the purposes of improved understanding, as well as illustrating some additional optional features of the downlight lamp.

Figure 4 also illustrates how the visible light reflector 111 may form part of a capping portion 410 of the lamp 100. The capping portion 410 may comprise a protruding edge 411, e.g., for abutting and/or supporting the baffle(s) 210.

The capping portion 410 may, for instance, further comprise one or more air inlet channel obscurers or blockers 412, each air inlet channel obscurer 412 being configured to partially shield or obscure the air inlet channel(s). This achieves a same effect as the baffle(s) 210, in interrupting a line of sight from outside the lamp 100 to the sterilization chamber through the air inlet channel(s). The air inlet channel obscurer(s) or blocker(s) can therefore be alternatively labelled baffles, louver or the like.

This technique is also advantageous when the sterilization device 150 comprises an ultraviolet light emitter, as the baffle(s) or louver will act to block at least some of the ultraviolet and/or blue light emitted by the sterilization device from escaping the downlight lamp 100.

Figure 4 illustrates how the baffle(s) 210 may be held or mounted on the second sleeve 192, e.g., to cover or overlap with the air outlet openings. Figure 4 also provides a clear view of how the baffles 210 may be configured and/or positioned to partially overlap one another, e.g., to complete block a line of sight to the air outlet channel(s) and/or air inlet channel(s) from a position external to the downlight lamp.

Another optional feature of the downlight lamp is a clamping portion 450. The clamping portion is configured to hold or support the downlight lamp to a supporting member, such as a recess or gap of a supporting member. Clamping portions are well known in the art, and could be replaced by alternative gripping or connecting means (e.g., screws, clipping mechanisms or the like).

The downlight lamp 100 may comprise a fan cover 165, which is disposed between the fan 160 and the sterilization chamber. In particular, the fan cover 165 defines a bound of the sterilization chamber, and may support the sterilization device 150 within the sterilization chamber.

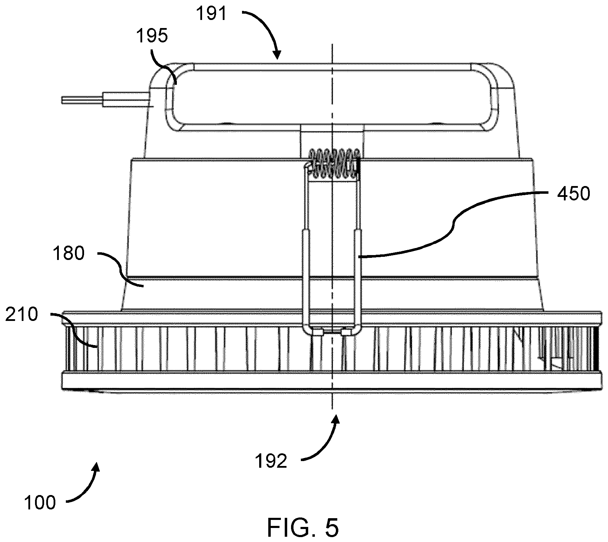

Figure 5 provides a side view of the downlight lamp, more clearly illustrating the optional one or more baffles 210 and the clamping portion 450.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A downlight lamp (100) for providing visible light illumination and air sterilization, the downlight lamp being adapted for mounting on a supporting member (410) and comprises:
a visible light arrangement (110) for emitting visible light out of the downlight lamp;
one or more air inlet channels (120) for receiving unsterilized air (125) into the downlight lamp, each air inlet channel comprising an air inlet opening (121) through which air is received into the downlight lamp;
one or more air outlet channels (130) for outputting sterilized air (135) from the downlight lamp, each air outlet channel comprising an air outlet opening (131) through which air is output from the downlight lamp;
a sterilization chamber (140) coupled to the one or more air inlet channels and the one or more air outlet channels;
a sterilization device (150) configured to sterilize air in the sterilization chamber; and
a fan (160) for moving air through the one or air inlet channels and out of the one or more air outlet channels via the sterilization chamber,
wherein any plane in which any air inlet opening lies makes an angle of between 60° and 120° to any plane in which any air outlet opening lies; and
wherein each air inlet opening (121) and each air outlet opening (131) are provided at a portion of the downlight lamp that protrudes from a side of the supporting member in the same direction as the visible light is emitted out of the downlight lamp.

2. The downlight lamp (100) of claim 1, wherein any plane in which any air inlet opening lies makes an angle of between 80° and 100°, and preferably between 85° and 95°, to any plane in which any air outlet opening lies.

3. The downlight lamp of claim 1 or 2, wherein the shape of each air inlet channel (120) and each air outlet channel (130) is curved.

4. The downlight lamp of any of claims 1 to 3, wherein each air inlet channel (120) is positioned between at least one air outlet channel (130) and the visible light arrangement (110).

5. The downlight lamp of any of claims 1 to 4, wherein each air outlet opening (131) is positioned to point away from each air inlet opening (121).

6. The downlight lamp of any of claims 1 to 5, wherein the visible light arrangement (110) comprises:
a visible light emitter (111) configured to generate and emit visible light; and
a visible light reflector (112) configured to direct any visible light generated by the visible light emitter out of the downlight lamp,
wherein the visible light reflector provides at least part of the boundaries of at least one air inlet channel and/or air outlet channel.

7. The downlight lamp of claim 6, further comprising:
a first sleeve (170) surrounding the visible light reflector, wherein the space between the first sleeve and the visible light reflector defines either: the one or more air inlet channels or the one or more air outlet channels; and
a second sleeve (180) surrounding the first sleeve, wherein the space between the first sleeve and the second sleeve defines the other of: the one or more air inlet channels or the one or more air outlet channels.

8. The downlight lamp of any of claims 1 to 7, wherein the fan (160) comprises a centrifugal fan.

9. The downlight lamp of any of claims 1 to 8, wherein the sterilization device (150) comprises an ultraviolet light emitter configured to irradiate the sterilization chamber (140) with ultraviolet light to sterilize air in the sterilization chamber.

10. The downlight lamp of claim 9, wherein the ultraviolet light emitter is configured to only emit ultraviolet light in directions that neither head toward the one or more air inlet channels nor toward the one or more air outlet channels.

11. The downlight lamp of any of claims 1 to 10, further comprising one or more baffles (210) positioned to at least partially shield the at least one air outlet opening.

12. The downlight lamp of any of claims 1 to 11, wherein the width (W2) of any of the air inlet opening (130) is greater than the width (W1) of any air outlet opening (120).

13. The downlight lamp of any of claims 1 to 12, wherein the sterilization device is positioned between the one or more air inlet channels and the fan.

14. The downlight lamp of any of claims 1 to 13, wherein the downlight lamp comprises:
a first end (191) for connecting to a lamp socket; and
a second end (192) at which visible light, emitted by the visible light arrangement, is output,
wherein each air inlet opening and each air outlet opening are positioned at the second end.

15. The downlight lamp of any of claims 1 to 14, wherein the visible light arrangement is configured to emit light having a wavelength greater than 380nm.
